# EUROPEAN PATENT APPLICATION

(11) **EP 2 851 088 A1**
(43) Date of publication of application: **25.03.2015**
(21) Application number: 14183927.4
(22) Date of filing: 23.06.2004
(51) Int. Cl.: A61K 39/385, A61K 39/095, A61K 39/40, A61K 47/48, C07H 1/00

(54) **Vaccines against group Y Neisseria meningitidis and meningoccal combinations thereof**

(30) Priority: 23.06.2003 US 480405 P
(62) Divisional of application: 04776938.5
(71) Applicant: Baxter International Inc., Deerfield, IL 60015 (US); Baxter Healthcare S.A., 8152 Glattpark (Opfikon) (CH)
(72) Inventor: Michon, Francis J., Bethesda, MD 20814 (US)
(74) Representative: Williams, Gareth Owen

(57) **Abstract**

This invention relates to modified meningococcal Y polysaccharides (GYMP), conjugates comprising the modified polysaccharides and a carrier, vaccines for the immunisation of warm-blooded animals, including humans, against Group Y Neisseria meningitidis, and to methods for producing these modified polysaccharides, conjugates and vaccines.

## Description

### TECHNICAL FIELD

The present invention relates to modified meningococcal Y polysaccharides (GYMP), conjugates comprising the modified polysaccharides and a carrier, vaccines for the immunisation of warm-blooded animals, including humans, against Group Y *Neisseria meningitidis,* and to methods for producing these modified polysaccharides, conjugates and vaccines.

### BACKGROUND OF THE INVENTION

Bacterial meningitis is a serious threat to global health. *Neisseria meningitidis* is a major cause of bacterial meningitis and sepsis. *Neisseria meningitidis* is encapsulated by polysaccharide capsules. *Neisseria meningitidis* isolates can be divided into 12 groups based on their chemically and antigenically distinct polysaccharide capsules,. Five of these groups A, B, C, Y and W135 are responsible for virtually all cases of bacterial meningitis and sepsis in humans.

The incidence of bacterial meningitis caused by group Y *Neisseria meningitidis* is increasing. Active laboratory-based surveillance conducted during the period 1989 to 1995 in a number of counties in the United States showed that the proportion of serogroup Y meningococcal disease increased dramatically from 0% in 1989 to 32.5% in 1995. During this period the overall incidence of meningococccal disease remained stable (Anonymous 1996, Serogroup Y meningococcal disease - Illinois, Connecticut, and selected areas, USA 1989-1996, Morb. Mortal. Rep. 45, 1010-1013).

Although bacterial meningitis caused by group Y *Neisseria meningitidis* can occur in children and young adults, it has a propensity to cause disease in the elderly and is more likely to cause pneumonia than other strains of bacterial meningitis. Polysaccharide vaccines have been used to prevent diseases caused by several serogroups of *Neisseria meningitidis.* However, these vaccines are not effective for the prevention of diseases caused by *Neisseria meningitidis* in certain sections of the population.

Polysaccharides are T-independent (thymus-independent) antigens. They have a number of immunological properties that are not encountered when inducing an immune response to proteins. These properties include no overt requirement for the presence of T-cells to induce an immune response, dominance of IgM, no memory induction or affinity maturation after immunisation, and poor immunogenicity in infants, the elderly and the immunocompromised. As a result, pure polysaccharide vaccines cannot be effectively used for these patients.

It was reported by Jennings in the 1970s (Bhattacharjee et al,. Can. J. Biochem. 54:1-8, 1976) that the serogroup Y polysaccharide is composed of equimolar proportions of N-acetylneuraminic acid and D-glucose and is partially O-acetylated.

It has been found that most group Y meningococcal isolates produce an O-acetyl positive (OA) polysaccharide in which the O-acetyl groups are distributed exclusively between the C-7 and C-9 of its sialic acid residues. This heterogeneity in O-acetyl group distribution, both in location and in concentration, complicates formulation of a polysaccharide conjugate.

### SUMMARY OF THE PRESENT INVENTION

It is an object of the present invention to provide a vaccine suitable for use in immunising against group Y *Neisseria meningitidis.*

The present invention provides a vaccine comprising at least one modified meningococcal Y polysaccharide or a fragment of modified meningococcal Y polysaccharide or a fragment of meningococcal Y polysaccharide. In the following text, the term "meningococcal Y polysaccharide" and forms thereof include modified meningococcal Y polysaccharide, a fragment of modified meningococcal Y polysaccharide and a fragment of meningococcal Y polysaccharide. These polysaccharides may be used, alone, to produce an antigenic composition which elicits a T-independent response.

In order to elicit a T-dependant immune response, typically the modified meningococcal Y polysaccharide is conjugated to a carrier protein. Thus, the present invention also provides a conjugated material comprising a modified meningococcal Y polysaccharide and a carrier protein and a method for producing the conjugated material.

The present invention also provides a method for producing the modified meningococcal Y polysaccharide. As the meningococcal Y polysaccharide contains O-acetyl groups that are distributed exclusively between the C-7 and C-9 of its sialic residues, the meningococcal Y polysaccharide can be modified by de-O-acetylation. Thus, the present invention also provides a process for the de-O-acetylation of the meningococcal Y polysaccharide. In one embodiment, base hydrolysis may be used to de-O-acetylate the polysaccharide.

The present invention also provides a process for fragmenting a de-O-acetylated meningococcal Y polysaccharide or an O-acetylated meningococcal Y polysaccharide. Mild acid hydrolysis is used in one embodiment to fragment the polysaccharide. Other methods for the cleavage of the glycosidic bonds of the polysaccharide include ozonolysis, sonication and base hydrolysis.

### BRIEF DESCRIPTION OF THE DRAWINGS

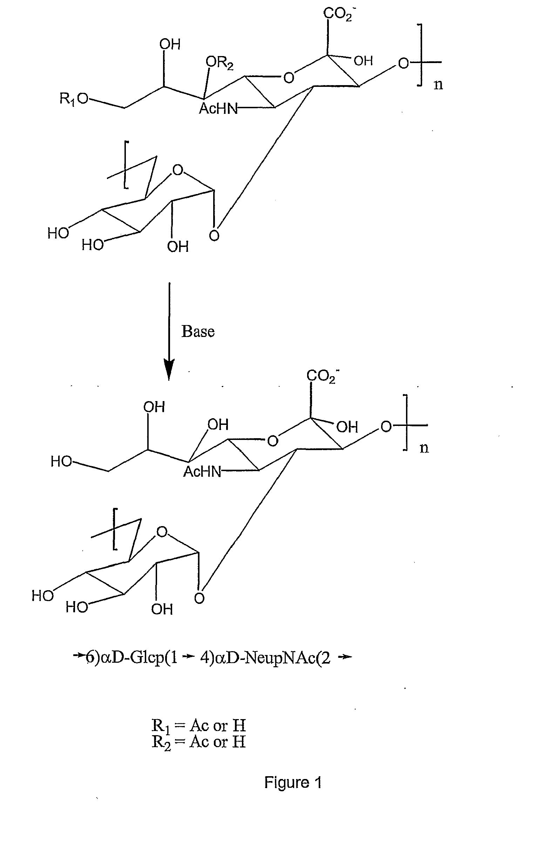

**Figure 1** shows the structure of the repeating unit the de-o- acetylated-group Y ("deOAc-Y") meningococcal polysaccharide, bottom and its preparation from native o-acetylated group Y ("OAc-Y") meningococcal polysaccharide, top.

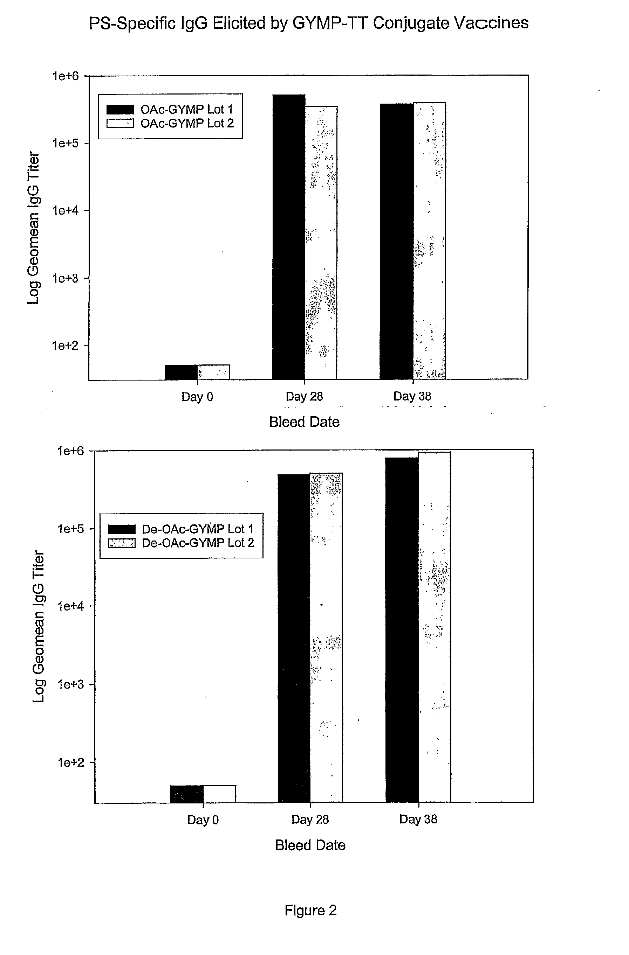

**Figure 2** shows the geometric mean of Y *Neisseria meningitidis* polysaccharide specific IgG titers elicited by OAc-Y (top) and deOAc-Y (bottom) *Neisseria meningitidis* polysaccharide-tetanus toxoid conjugates after one or two injections in female Swiss Webster mice, measured as described in Example 3.

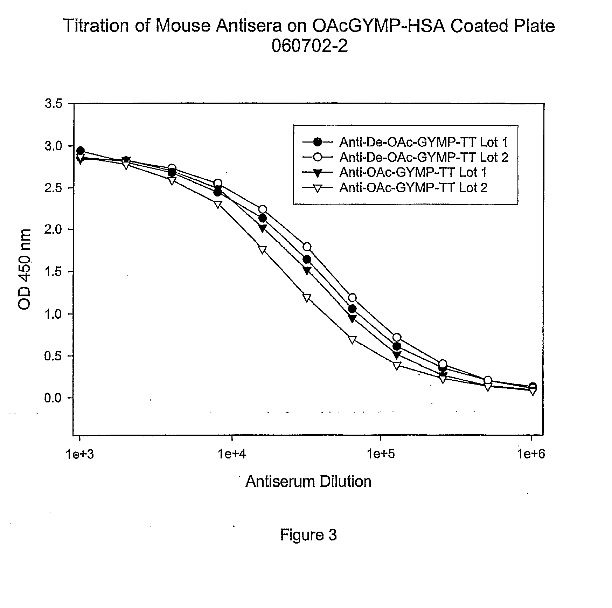

**Figure 3** shows the ELISA titration of mouse antisera induced with OAc-Y and deOAc-Y *Neisseria meningitidis* polysaccharide-tetanus toxoid conjugates on OAc-Y *Neisseria meningitidis* polysaccharide-HSA coated plates at 38 days, as measured in Example 3.

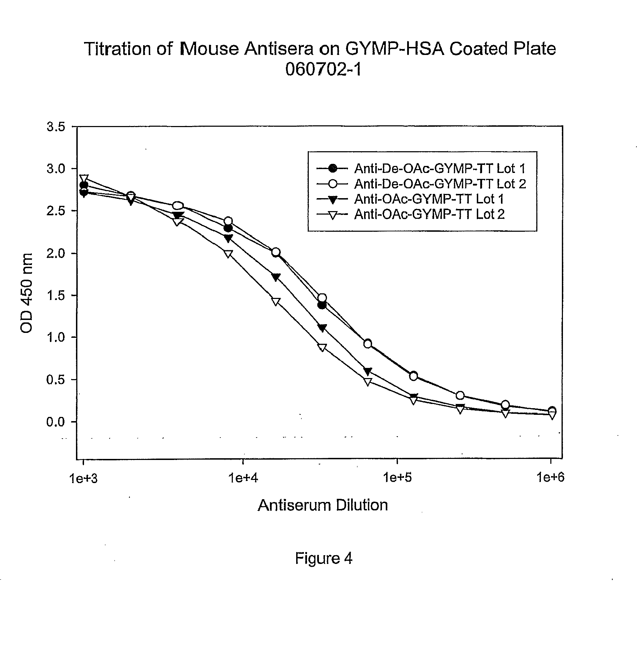

**Figure 4** shows the ELISA titration of mouse antisera induced with OAc-Y and deOAc-Y *Neisseria meningitidis* polysaccharide-tetanus toxoid conjugates on deOAc-Y *Neisseria meningitidis* polysaccharide-HSA coated plates at 38 days, as measured in Example 3.

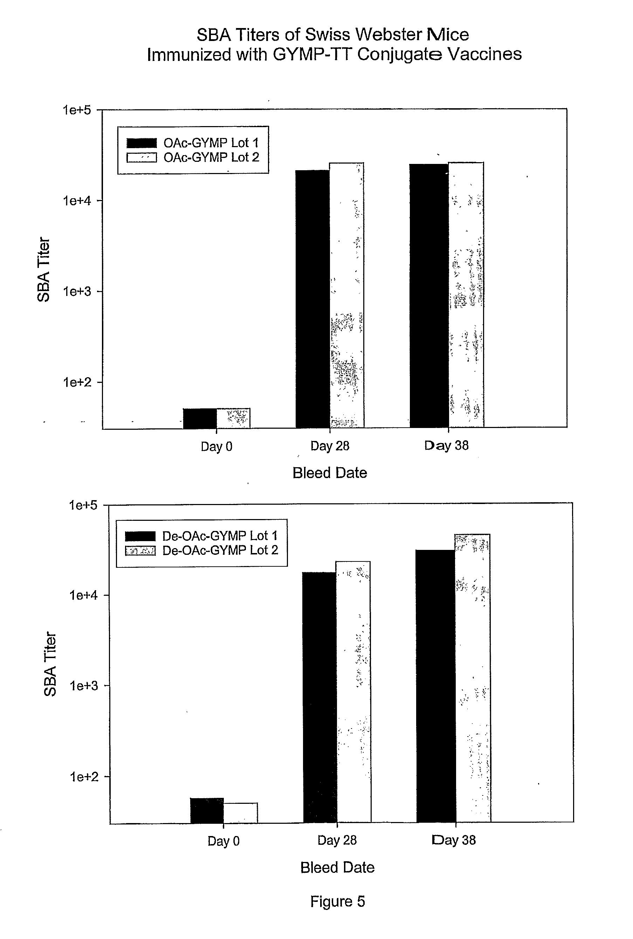

**Figure 5** shows serum bactencidal activity titers, measured with rabbit complement and OAc-Y *Neisseria meningitidis* strain 3790 of mouse antisera induced with OAc-Y (top) and deOAc-Y (bottom) *Neisseria meningitidis* polysaccharide-tetanus toxoid conjugates, as measured in Example 3.

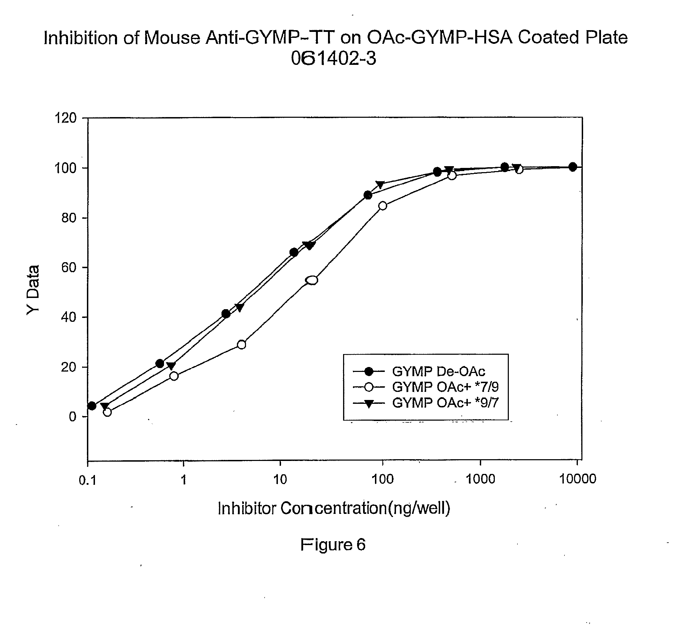

**Figure 6** shows the inhibition of binding of deOAc-Y *Neisseria meningitidis* polysaccharide conjugate antiserum on a microtiter plate coated with OAc-GYMP-HSA conjugate relative to OAc-Y with greater amounts of o-acetylation at carbon 9 (*9/7) or carbon 7 (*7/9) and deOAc-Y *Neisseria meningitidis* polysaccharides, as measured in Example 4.

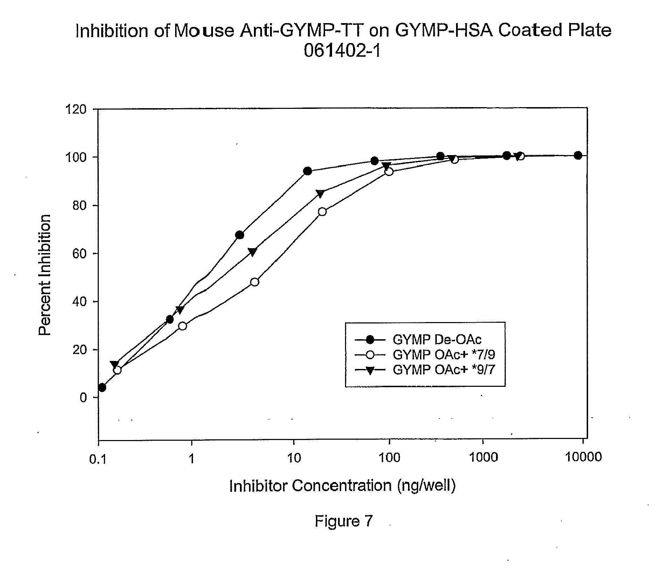

**Figure 7** shows the inhibition of binding of deOAc-Y *Neisseria meningitidis* polysaccharide conjugate antiserum on a microtiter plate coated with DeOAc-GYMP-HSA conjugate relative to OAc and deOAc-Y *Neisseria meningitidis* polysaccharides, as measured in Example 4.

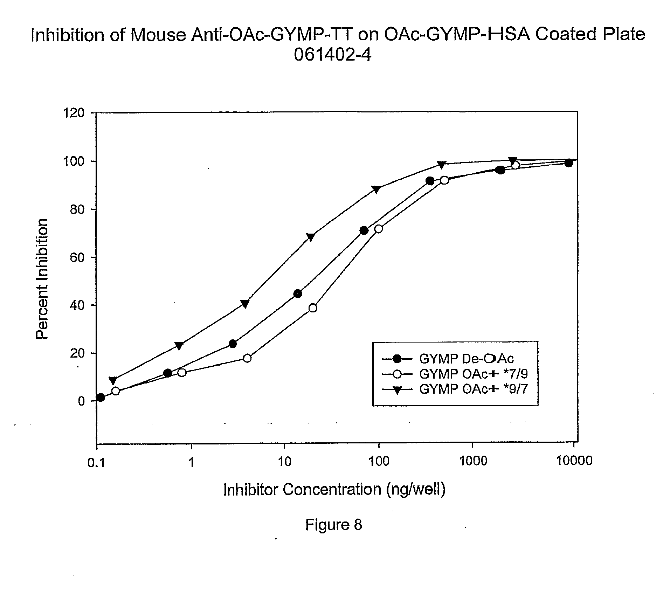

**Figure 8** shows the binding inhibition of OAc-Y *Neisseria meningitidis* polysaccharide conjugate antiserum on a microtiter plate coated with OAc-Y *Neisseria meningitidis* polysaccharide-HSA conjugate relative to OAc-Y and deOAc-Y *Neisseria meningitidis* polysaccharides, as measured in Example 4.

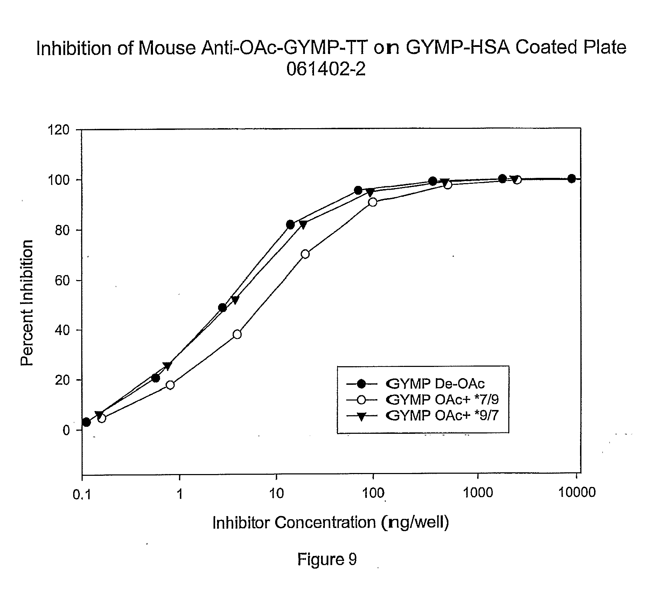

**Figure 9** shows the binding inhibition of OAc-Y *Neisseria meningitidis* polysaccharide conjugate antiserum on a microtiter plate coated with deOAc-Y *Neisseria meningitidis* polysaccharide-HSA conjugate relative to OAc-Y and deOAc-Y *Neisseria meningitidis* polysaccharides, as measured in Example 4.

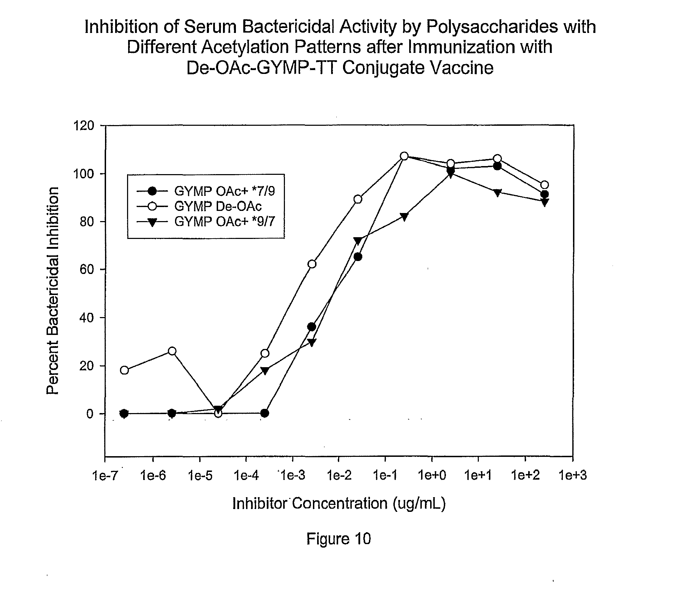

**Figure 10** shows the specificity of functional antibodies induced by OAc-Y or deOAc-Y *Neisseria meningitidis* polysaccharide conjugates as measured by competitive inhibition of SBA (using rabbit complement) to an OAc-Y *Neisseria meningitidis* polysaccharide strain (3790) with soluble Y *Neisseria meningitidis* polysaccharide inhibitors, using mouse anti-dOAc-Y *Neisseria meningitidis* polysaccharide conjugate antiserum, as measured in Example 4.

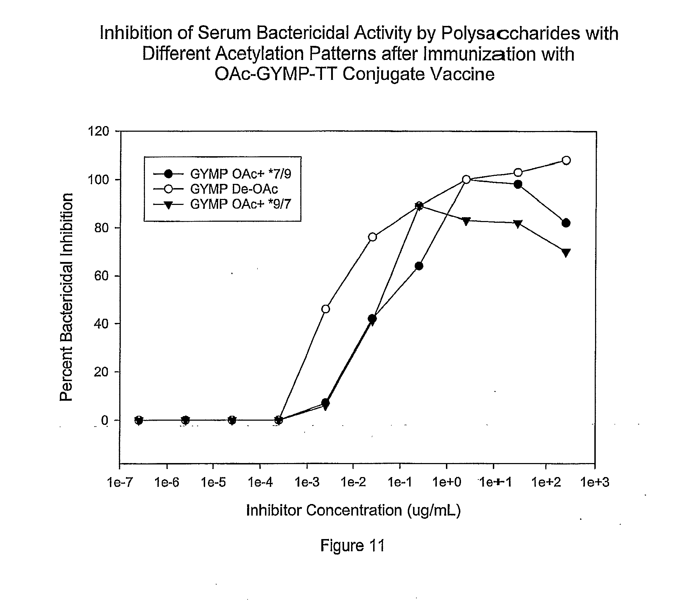

**Figure 11** shows the results of competitive inhibition of SBA using mouse anti-OAc-Y *Neisseria meningitidis* polysaccharide conjugate antiserum, as measured in Example 4.

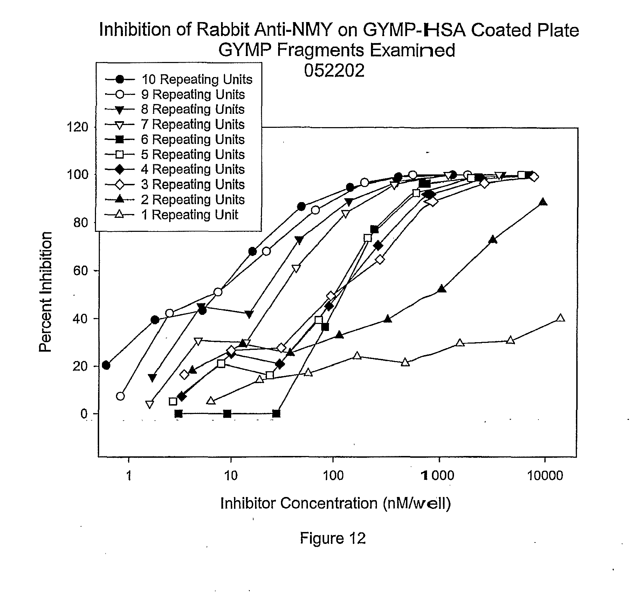

Figure 12 shows an ELISA inhibition of a rabbit anti-whole cell OAc-Y *Neisseria meningitidis* polysaccharide antibody binding to a deOAc-Y *Neisseria meningitidis* polysaccharide-HSA coated plate, with deOAc-Y *Neisseria meningitidis* oligosaccharides ranging from 1 to 10 repeating units, as measured in Example 5.

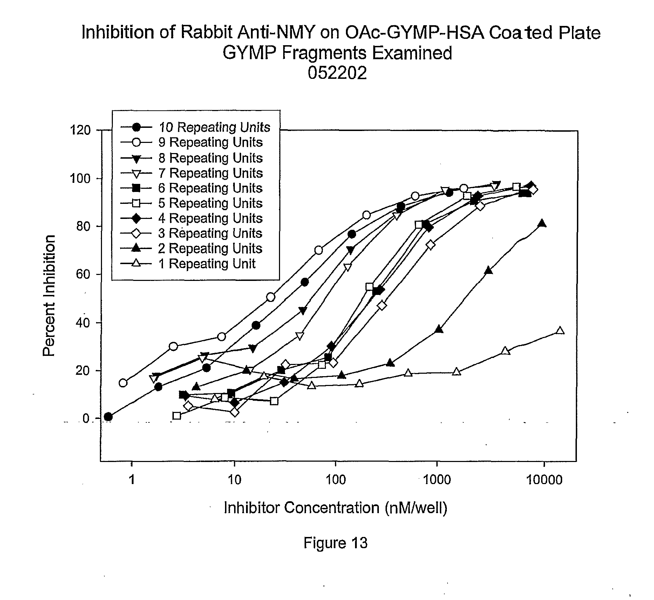

**Figure 13** shows an ELISA inhibition of a rabbit anti whole cell OAc-Y *Neisseria meningitidis* polysaccharide antibody binding to an OAc-Y *Neisseria meningitidis* polysaccharide-HAS coated plate, with deOAc-Y *Neisseria meningitidis* oligosaccharides ranging from 1 to 10 repeating units, as measured in Example 5.

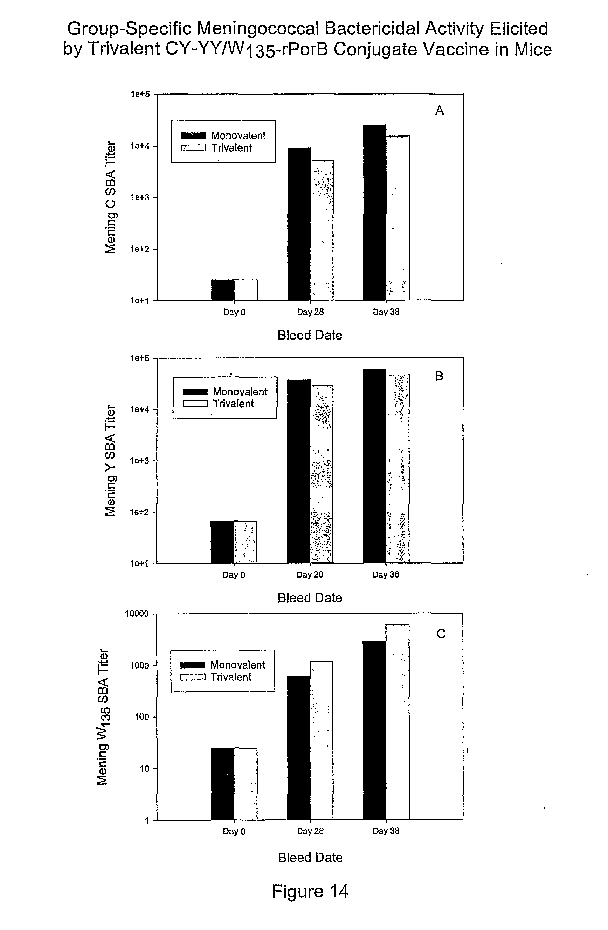

**Figure 14** shows the results of tests relating to the potency of a deOAc-Y *Neisseria meningitidis* polysaccharide and tetanus toxoid conjugate in combination with a deOAc-C *Neisseria meningitidis* polysaccharide and tetanus toxoid conjugate and a deOAc-W *Neisseria meningitidis* polysaccharide and rPorB conjugate in Swiss Webster mice, as measured in Example 6.

### DETAILED DESCRIPTION OF THE PREFERRED EMBODIMENTS OF THE PRESENT INVENTION

In accordance with the present invention, a meningococcal Y polysaccharide may be de O-acetylated using base hydrolysis. Base hydrolysis may be performed by heating a solution of the polysaccharide with a base.

Suitable bases include strong bases such as alkali metal hydroxides or alkali metal alkyl-oxides. Specific examples of suitable bases include NaOH, KOH, LiOH, NaH, NaOMe, and KO*t*Bu.

The concentration of the base used will, of course, depend on the nature of the base used, including considerations such as the dissociation constant of the base. The person of ordinary skill in the art would be able to readily determine what concentration of base would be appropriate. When the base is NaOH a suitable concentration for the base when mixed with the polysaccharide is 0.1 to 10 N. In various exemplary embodiments, an appropriate concentration would be between 1 to 5 N, such as 2 N, 3 N or 4 N.

Suitable reaction temperatures will depend on the base used, and the concentration chosen. Typically, a reaction temperature of from 50 to 100 °C, is suitable-Particularly, temperatures between 75 °C and 85 °C, such as about 80 °C, are suitable.

The reaction time will depend on a number of factors such as the base used, the concentration of the base and the reaction temperature. Generally, the reaction time will increase with decreasing base concentration or temperature. Suitable reactions times are typically 10 to 25 hours, and more typically 15 to 20 hours, for example 16, 17, 18 or 19 hours.

The person of ordinary skill in the arts of carbohydrate chemistry will appreciate that the above parameters may be manipulated in order to optimize a particular parameter, and to optimize yield. By doing so, commercially relevant variables such as chemical consumption (base), temperature, and time can be optimized for production in a particular system. For example, one might wish to minimize the time required to produce the de-o-acetylated Y *Neisseria meningitidis* polysaccharide, and thus choose to use the upper portion of the preferred ranges for base concentration and temperature. The examples provided in this description illustrate production of these polysaccharides on a relatively small scale, and the above parameters may be varied within the suggested ranges for adaptation to larger scale production systems.

The degree of de-O-acetylation is achieved by this process is complete, as measured by H-NMR spectroscopy. Optionally, the de-O-acetylated polysaccharide may be subjected to acid hydrolysis. This step is used to break down the large molecular polysaccharide into fragments of smaller Mw, providing more individual group Y meningococcal polysaccharide epitope presenting molecules per gram of starting material.

Acid hydrolysis may be performed by agitating a solution of the polysaccharide with an acid or a buffer-forming compound. Suitable acids or buffer-forming compounds include alkali metal acetates, such as sodium acetate, which buffer over an acidic pH between about 3-6. As those skilled in the chemical arts will appreciate, other salts of weak acids may be used to create a suitable buffer in this pH range. Buffers are particularly useful in the methods of the invention, as they maintain a source of hydrogen ions (H⁺) at a relatively constant concentration over the entire period of the reaction.

The concentration of the acid or buffer-forming compound used will, of course, depend on the nature of the acid or buffer-forming compound used. The person of ordinary skill in the art would be able to readily determine what concentration of acid and buffer-forming compound would be appropriate. A suitable concentration, when sodium acetate is mixed with the polysaccharide, is 0.01 to 1 N, for example 0.05 to 0.5 N, such as 0.1 N sodium acetate.

Suitable reaction temperatures will again depend on the acid or buffer-forming compound used. Typically, a reaction temperature of from 50 to 100 °C, more typically from about 65 to 80 °C such as about 70 °C is suitable.

In various embodiments of the invention, the reaction mixture may be agitated. The rate of agitation will depend on a number of factors such as the acid or buffer-forming compound used, the concentration of the acid or buffer-forming compound, the type of agitator used and the reaction temperature. If the reaction vessel is rotationally shaken, suitable rates of agitation are typically 50 to 100 rpm, for example 65 rpm.

The de-O-acetylated polysaccharide, regardless of whether or not it has been subjected to acid hydrolysis, may optionally undergo re-N-acetylation. This step ensures that all free primary amines that may have been generated during the base hydrolysis are re-N-acetylated.

Re-N-acetylation may be performed using acetic anhydride. Typically, this step takes place in a neutral or basic solution, for example the pH of the solution may be from 7 to 13, such as from 7 to 9, for example 8. Other alternative chemical agents for re-N-acetylation include of acetyl chloride, pentafluorophenyl acetate or 4-nitrophenyl acetate. Use of these agents is known in the chemical arts.

Suitable reaction temperatures will depend on factors such as the pH of the reaction mixture and the nature of the reagent used. Typically, the reaction takes place at about room temperature, such as 15 to 35 °C, and more typically from 20 to 25 °C.

In one embodiment of the invention, the process for preparing a de-O-acetylated meningococcal Y polysaccharide fragment comprises:
a) providing an at least partially purified meningococcal Y polysaccharide;
b) base hydrolysis of the polysaccharide;
c) acid hydrolysis of the product of step (a).

Optionally, the product of step (b) may be re-N-acetylated.

The use of a strong base treatment to purify polysaccharide is described in US Patent No. 6248570, hereby fully incorporated herein by reference.

An O-acetylated meningococcal Y polysaccharide can be fragmented using the acid hydrolysis process described above.

The de-O-acetylated polysaccharide, regardless of whether or not it has been subjected to acid hydrolysis, may optionally undergo the N-acetylation process described above.

The meningococcal Y polysaccharide used in the processes described above may be isolated form the meningococcal bacteria prior to use. Any method of isolation known in the art may be used (see US Patent No. 6248570.) Suitable methods of isolation include centrifugation followed by ultra-filtration. Confirmation of the identity and quantification of the isolated polysaccharide may be accomplished by H-NMR spectroscopy, GC-MS analysis of the sugar constituents, ELISA using typing antiserum, or other methods known to those of skill in the art.

The polysaccharides that have been modified using those processes described above that include an acid hydrolysis step typically have a low molecular weight. As used herein, the term "low molecular weight" means a molecular weight of less than 100 kDa, such as from 5 to 50 kDa, for example from 10 to 20 kDa, as measured by size exclusion chromatography coupled to mutiangle laser light scattering (SEC-MALLS).

Both the de-O-acetylated polysaccharide and the O-acetylated polysaccharide can be activated by the generation of reducing groups by oxidation. Suitable methods for generation of the reducing groups include limited oxidative cleavage by periodate (or a related reagent such as paraperiodic acid, sodium metaperiodate and potassium metaperiodate) which produces aldehydic termini.

Without limiting themselves to any particular theory, applicants propose that the aldehyde groups are selectively introduced at C-8 into the sialic acid exocyclic side chains of the polysaccharides. In other words, it is thought that oxidation occurs between the C-8 and C-9 positions.

The activated polysaccharide groups are suitable for conjugation with a suitable carrier protein. Thus, the present invention also provides a conjugated product containing a de-O-acetylated Y *Neisseria meningitidis.* polysaccharide or a fragment thereof conjugated to a suitable carrier protein. As the O-acetylated Y *Nesseria meningitides* polysaccharide may be activated, the disclosed methods also provide a O-acetylated Y *Nesseria meningitides* polysaccharide or a fragment thereof conjugated to a suitable carrier protein.

Any suitable protein can be used as a carrier protein. To be suitable for use in the present invention, carrier proteins must be safe to administer to the mammal to be treated, for example infants, and immunologically effective. Safety requirements include an absence of primary toxicity and minimal risk of allergic reactions.

Suitable carrier proteins include bacteria toxins and toxoids. Examples of suitable bacteria toxins and toxoids include, but are not limited to, diphtheria, tetanus, pseudomonas, staphylococcus, streptococcus, PorB such as rPorB (as described in US Patent No. 5439808) and all others derivatives thereof, pertussis and enterotoxigenic bacteria, including *Escherichia coli* toxins or toxoids. In addition to whole toxins and toxoids, fragments or portions of protein toxoids which exhibit the appropriate immunostimulatory effect may also be used. For instance, fragment C of tetanus toxoid may be used as a carrier. The toxin or toxoid used may be derived from its native source (e.g., *Bortadella pertussis* bacteria for pertussis toxoid,) or may be produced recombinantly.

By choosing an appropriate carrier protein is chosen, a "carrier effect" effect is achieved. The "carrier effect" allows the Y *Neisseria meningitidis* polysaccharide to become more immunogenic due to its attachment to the carrier than if the Y *Neisseria meningitidis* polysaccharide were presented alone.

An enhanced response may be obtained if the mammal to be immunised has previously been immunised with the carrier alone. Infants are routinely immunised with tetanus and diphtheria toxoids. Thus, it can be expected that the use of these toxoids in vaccines intended to immunise infants against Y *Neisseria meningitidis* will produce an enhanced effect. The achievement of the "carrier effect" may be verified by monitoring the immunological response of animal models or humans to the challenge with the polysaccharide-carrier protein conjugate.

Some bacterial toxins, such as tetanus and diphtheria toxins, are composed of two or more proteins. One of these proteins has a strong affinity for binding to mammalian cell surfaces. Again, without wishing to be bound by theory, it is possible that the use of a protein with this strong binding ability may more effectively initiate a response from the immune system.

The carrier protein may be a native toxin or detoxified toxin (toxoid). Alternatively, proteins that have been genetically modified by mutational techniques to provide a protein that is antigenically similar to the toxin but non-toxic may be used. Proteins modified in this manner are known as "cross reacting materials" or CRMs. One such material that may be used in the present invention is CRM₁₉₇. CRM₁₉₇ is based on the native diphtheria toxin. It has a single amino acid change, namely glycine 52 in the wild type toxin is replaced with glutamic acid in CRM₁₉₇, and is immunologically indistinguishable from the native diphtheria toxin.

In one aspect of the invention, the bacterial toxin or toxoid is tetanus toxin or toxoid or diphtheria toxin or toxoid.

Thus, the present invention provides a conjugated product comprising a de-O-acetylated Y *Neisseria meningitidis* polysaccharide or an O-acetylated Y *Neisseria meningitidis* polysaccharide conjugated to tetanus toxin or toxoid or diphtheria toxin or toxoid.

When a native toxin is used, conjugation to the Y *Neisseria meningitidis* polysaccharide may reduce the toxicity of the native toxin. However, it is likely that remaining toxicity levels would be too high. Therefore, further detoxification may be necessary. Any suitable method known in the art may be used to detoxify the protein. Conventional methods include the use of formalin, which reacts with free amino groups on the protein.

Alternatively, the native toxin may be detoxified, for example with formalin, to produce a toxoid before conjugation to Y *Neisseria meningitidis* polysaccharide.

A conjugate of an Y *Neisseria meningitidis* polysaccharide and a suitable carrier protein can be obtained by contacting an activated polysaccharide with a suitable carrier. Usually, an excess of polysaccharide over the protein carrier is used, typically an excess of 2 to 3 times by weight. The ratio of polysaccharide to protein carrier can be varied by one of skill in the art to achieve different numbers of antigenic polysaccharide moieties per conjugate. For higher ratios of polysaccharide moieties per conjugate, one would start with more polysaccharide per carrier protein; to obtain lower ratios, one would start with less polysaccharide per carrier protein.

Reductive amination is a preferred mode of coupling, as described in US Patent No 4356170, hereby incorporated fully herein by reference. Thus, preferably the activated polysaccharide and the carrier are contacted in the presence of a reductive coupling agent. Suitable coupling agents include reducing agents such as cyanoborohydride ions or their equivalent, and may be provided in the form of a suitable salt such as sodium cyanoborohydride. Any reducing agent that will not reduce the reducing ends of interests nor adversely affect the carrier protein or the polysaccharide may be used as a coupling agent. For example, pyridine borane is another selective reducing agent suitable for this purpose. Other suitable reducing agents will be apparent to those of skill in the art.

Selective reductive coupling agents are though to act as a mild selective reducing agent of the Schiff base intermediate that is formed between the carbonyl groups of the polysaccharide and the amino groups of the carrier protein. It is thought that these ions have a secondary effect of slowing reduction of any active aldehyde groups remaining on the polysaccharide after conjugation has occurred.

The coupling agent does not normally form part of the conjugated product, but is rather consumed during the coupling reaction. The conjugates may then be purified to remove remaining coupling agent and any reaction by-products. Preferably, the purified conjugates do not contain potentially toxic linking agents such as adipic dihydrazide or *p*-nitro-phenyl-ethylamine, which have previously been used to conjugate carbohydrates to proteins.

The conditions under which the activated polysaccharide and the carrier are contacted will depend on the nature of the carrier. For example, when the carrier is tetanus toxoid, the activated polysaccharide and the carrier may be contacted at an approximately neutral pH, such as about 7.4, at a temperature of from about 20 to 50 °C, such as 30 to 40 °C, for example 37 °C.

Once the coupling reaction is complete, any residual aldehyde groups on the polysaccharides may be capped. This will reduce any remaining aldehyde groups on the meningococcal polysaccharide chains which were generated during the activation step, but which were not utilized to conjugate the polysaccharide to the carrier protein. Suitable capping agents include NaBH₄, or other reducing agents know to those of skill in the art which are capable of reducing the remaining aldehyde groups to alcohol groups.

The de-O-acetylated Y *Neisseria meningitidis* polysaccharide produced by the methods of the invention may be utilized as an antigen in a vaccine preparation, although this preparation will not produce a T-dependent immune response. A conjugate of an Y *Neisseria meningitidis* polysaccharide and a suitable carrier protein is more preferably be used as a vaccine or in a vaccine composition for use in immunisation against group Y *Neisseria meningitids.* Thus, in some preferred embodiments the vaccine of the invention comprises a conjugate comprising a de-O-acetylated polysaccharide. As shown in the data of the examples, the de-O-acetylated polysaccharide conjugate is capable of eliciting an immune response in animals which produces antibodies cross-reactive with the O-acetylated polysaccharide.

In accordance with ordinary formulation practice, the polysaccharide or conjugate is rendered stable and checked for absence of pynogenicity before or after formulation as a vaccine. In one aspect, the present invention provides a vaccine comprising a de-O-acetylated Y *Neisseria meningitidis* polysaccharide conjugated to tetanus toxin or toxoid or diphtheria toxin or toxoid. The vaccine may contain components that are conventionally present in vaccine compositions. For example the vaccines may comprise one or more of a suitable carrier medium, excipient(s), diluent(s) or adjuvant(s).

Suitable carriers include physiological sodium phosphate-buffered saline (pH 7.4). An example of a carrier with an adjuvant would be 0.125 M aluminium phosphate gel suspended in sodium phosphate-buffered saline at pH 6. Other pharmaceutically acceptable carriers suitable for use in vaccines are known in the art and could be used in the present invention. Suitable adjuvants include aluminium hydroxide, and other adjuvants known to those of skill in the art such as, for example, generally immunogenic nucleic acid or peptide compounds.

The vaccines of the present invention can be administered by any appropriate method. For example, they may be administered by injection, for example intramuscular or sub-cutaneous injection. Needleless transdermal, Intranasal or mucosal administration, with a suitable carrier, may also be utilized

Typically, vaccines of the invention contain from 5 to 100 µg, preferably from 10 to 50µg of the conjugated material. The exact dosage may be determined by routine dose/response experimentation. The vaccine may be given as a single dosage or as two or more, for example three, smaller dosages.

The conjugated products of the present invention can also be used in vaccines for use in immunisation against more that one strain of *Neisseria meningitidis.* For example, the conjugated products of the invention can be used in vaccines intended to provide immunisation against one or more of *Neisseria meningitidis* A, C and W135 in addition to *Neisseria meningitidis* Y. The conjugated products can also be used in vaccines intended to provide immunisation against other diseases in addition to *Neisseria meningitidis.* For instance, the conjugates of the invention may be combined with a *Haemophilus influenza* conjugate to produce a combination Hib-meningococcal vaccine.

The use of the conjugated products of the present invention as vaccines produces a "carrier effect". The Y *Neisseria meningitidis* polysaccharide becomes more immunogenic due to its attachment to a stronger antigen as a carrier compared to if the Y *Neisseria meningitidis* polysaccharide were presented alone. In particular, the vaccines of the present invention can be used to elicit effective levels of anti-*Neisseria Meningitidis* Y antibody formation in young mammals including humans and are much less age dependent than vaccines comprising an unconjugated polysaccharide.

When the carrier protein is a bacterial toxin or toxoid of a disease against which the recipient of the vaccine of the invention has been vaccinated, for example tetanus or diphtheria, the desired immunity to the carrier toxin or toxoid may be achieved at the same time as immunity against *Neisseria meningitidis* Y.

Alternatively, if the recipient of the vaccine containing a conjugated product of the present invention has previously been vaccinated against the disease associated with the carrier toxin or toxoid, enhanced production of antibodies against *Neisseria meningitidis* Y may be achieved through a "booster" effect.

The present invention also provides for the use of a de-O-acetylated Y *Neisseria meningitidis* polysaccharide and/or an O-acetylated Y *Neisseria meningitidis* polysaccharide as described above in the manufacture of a vaccine for use in immunisation against group Y Neisseria meningitidis.

The present invention also provides for the use of a conjugated product comprising a de-O-acetylated Y *Neisseria meningitidis* polysaccharide and a suitable carrier such as tetanus toxin or toxoid or diphtheria toxin or toxoid and/or a conjugated product comprising an O-acetylated Y *Neisseria meningitidis* polysaccharide and a suitable carrier such as tetanus toxin or toxoid or diphtheria toxin or toxoid as described above in the manufacture of a vaccine for use in immunisation against group Y *Neisseria meningitidis.*

The present invention also provides the use of a de-O-acetylated Y *Neisseria meningitidis* polysaccharide and/or an O-acetylated Y *Neisseria meningitidis* polysaccharide as described above as a vaccine for use in immunisation against group Y Neisseria meningitidis.

The present invention also provides for the use of a conjugated product comprising a de-O-acetylated Y *Neisseria meningitidis* polysaccharide and a suitable carrier such as tetanus toxin or toxoid or diphtheria toxin or toxoid and/or a conjugated product comprising an O-acetylated Y *Neisseria meningitidis* polysaccharide and a suitable carrier such as tetanus toxin or toxoid or diphtheria toxin or toxoid as described above as a vaccine for use in immunisation against group Y *Neisseria Meningtidis.*

The present invention is illustrated by the following non-limiting Examples.

### EXAMPLE 1: EVALUATION OF O-ACETYL STATUS OF POLYSACCHARIDES FROM VARIOUS MENINGOCOCCAL Y STRAINS

### Preparation of native polysaccharides:

Meningococcal Y strains S1975, S225, S3536, and S3790 were kindly provided by Dr Carl Frasch (CBER/FDA, Bethesda, MD). Strain Y Slaterus was provided by Dr Francoise Collins (LCDC, Ontario, Canada). The strains were grown in shake flasks under agitation at 37 °C in a medium containing glucose and yeast extract. Cultures were harvested by centrifugation at 8000 rpm and supernatant was collected and sterile filtered through 0.22 µm filter units.

The microfiltered culture supernatants were concentrated by ultrafiltration using a filter device with a Biomax 300 kDa Pellicon membrane (0.5 m²) (Millipore Corp., Bedford, Mass. USA). The concentrated retentate was diafiltered 12 times against 1 M NaCl, then 10 times against deionized (DI) water and freeze dried. The high molecular weight purified "native" polysaccharides were analysed by GC-MS for sugar composition and by H-NMR spectroscopy at 500 MHz for estimation of O-acetyl content and location.

Table 1 summarizes the results of the GC-MS and H-NMR analysis.

**Table 1: Physicochemical characteristics of the polysaccharides**

| **Strain** | **Glc** | **NeuAc** | **OAc** | **OAc (C-7)^{ab}** | **OAc (C-9)^{ab}** |
|---|---|---|---|---|---|
| | **Mole/repeat** | **Mole/repeat** | **Mole/repeat** | **(Mole)** | **(Mole)** |
| S1975 | 1 | 1 | ND | - | - |
| S225 | 1 | 1 | ND | - | - |
| S3536 | 1 | 1 | 0.52 | 0.46 | 0.05 |
| S3790 | 1 | 1 | 0.90 | 0.80 | 0.10 |
| Slaterus | 1 | 1 | 0.93 | 0.32 | 0.61 |

| | | | | | |
|---|---|---|---|---|---|
| ^{a} The OAc group is solely contained on the NeuAc moiety of the repeating unit. ^{b} The OAc location and relative amount to sialic acids is based on the H-NMR signals assigned to H-7 (d, 5.04 ppm) and H-9 (d, 4.38 ppm), H-9 (dd, 4.15) resonances relative to H-1 α of the glucose moiety. The percentage (or moles) of OAc groups is also based on the assignments of signals at 2.14 ppm to CH₃(OAc) relative to signals at both 2.04 ppm to CH₃ (NAc) of OAc negative and/or O-acetylated at C-9 NeuAc residues and 1.96 to CH₃ of NeuAc residues O-acetylated at C- 7. ND: not detected | | | | | |

As can be seen from this table, there is a significant amount of strain-to-strain heterogeneity relative to the O-acetyl content of their capsular polysaccharides as well as the location of these O-acetyl groups whenever they are present. For example, although strain S3790 and Slaterus have roughly the same OAc content, the percentage of these groups (C-7 versus C-9) on their sialic acid residues is completely different. Polysaccharide from strain S3790 has OAc groups almost solely on one position (C-7) of its sialic acids, whereas polysaccharide from Slaterus has them predominately on C-9.

### EXAMPLE 2: PURIFICATION OF GROUP Y MENINGOCOCCAL POLYSACCHARIDE (GYMP)

### Preparation of dOA GYMP:

### Polysaccharide capture by UF with a 300 kDa MWCO membrane;

Approximately 13L of cell-free microfiltered fermentation permeate was concentrated by ultrafiltration to approximately 1 liter using a filter device with a Pellicon Biomax 300 kDa membrane (0.5 m²) (Millipore Corp., Bedford, Mass. USA). The concentrated retentate was diafiltered 12 times against 1 M NaCl and then 10 times against DI water. It was further concentrated to approximately 0.2L and collected.

### Base Hydrolysis of the polysaccharide:

The 300 kDa retentate solution (ca 5mg PS/mL) was adjusted to a final concentration of 2N NaOH and placed in an oven set to 80 °C for 16-18 hrs. After the reaction mixture had cooled off to less than 50 °C, it was diluted into 10L of DI water. After concentration through a 30 kDa MWCO Pellicon membrane, the concentrated retentate was diafiltered 12 times against 1M NaCl and then 10 times against DI water. It was further concentrated to approximately 0.2L and collected.

### Acid Hydrolysis of the dOA GYMP:

The retentate solution was transferred to a Teflon reaction vessel and sodium acetate (NaOAc) was added to a final concentration of 0.1 N. The reaction mixture was adjusted to pH 5 using 6N HCl and placed in a water bath set to 70 °C. It was shaken at 65 rpm until the polysaccharide reached a target MW of 20,000 daltons as measured by SEC-MALLS (Size exclusion chromatography coupled to multi-angle laser light scattering) using a Superose 12 (Pharmacia) column.

### Re-N-Acetylation of the fragmented dOA polysaccharide:

The pH of the solution was adjusted to 8 with 6N HCl solution, and acetic anhydride was then added dropwise at room temperature to a final concentration of 0.8 M acetic anhydride. 5N NaOH was used to keep the reaction mixture pH between 7 and 9. After completion of the reaction, the pH of the reaction mixture was increased to 13, and the mixture stirred for an additional 1.5 hr. The reaction pH was then adjusted to pH 8 with 6N HCl solution. The reaction mixture was poured into 4L of 1M NaCl, concentrated to approximately 1L using a device having a Pellicon Biomax 100 kDa membrane (0. 5 m²) and the permeate collected. The 100K final permeate was concentrated by UF to approximately 1 liter using a Biomax 5K Pellicon membrane (0.5 m²). The concentrated retentate was diafiltered 10 times against DI water, then concentrated to approximately 0.2L and collected.

### Preparation of OA GYMP:

The purification process for OA GYMP was similar to the one described above for the dOA polysaccharide with the exception that the alkaline degradation (2N NaOH) step that was omitted from the process. The GYMP obtained from this process had ca. 0.6 M of O-acetyl group per repeating units (or 60% relative to the N-acetyl group of sialic acid) equally distributed between the C7 and the C9 position of their sialic acids.

### Activation of GYMP prior to conjugation:

The low molecular weight (LMW) OA and dOAc polysaccharides (10-20 kDa) were oxidized with sodium metaperiodate to selectively introduce aldehyde groups at C-8 into their sialic acid exocyclic side chains (oxidation between C-9 and C-8).

### Preparation of Group Y Meningococcal Polysaccharide (GIMP) Conjugates

### Conjugation of meningococcal Y polysaccharides to tetanus toxoid:

Periodate-oxidized LMW GYMP Polysaccharides (25 mg) and tetanus toxoid (TT) (10 mg) (Serum Statens Institute, Copenhagen, Denmark) were dissolved in 0.5 ml of 0.2M phosphate buffer (pH 7.4). Sodium cyanoborohydride (5mg) was added, and the mixture was incubated at 37 °C for 1 day. After conjugation was complete, capping of residual aldehydes was carried out by adding very slowly in an ice-bath 40-60 µl of freshly prepared 100 mg/mL solution of NaBH₄ in 1mM NaOH with the pH of the solution maintained between 7 to 9 with 1M acetic acid.

The resulting conjugate was purified by ultrafiltration using a membrane of 100 kDa MWCO and a tangential flow filtration (TFF) system (Millipore) against 1 M NaCl, then against saline in a diafiltration mode.

Bulk conjugates were stored at 2-4 °C before being formulated.

### EXAMPLE 3: POTENCY OF GYMP CONJUGATES IN MICE

### Immunizations:

4-6 weeks old female Swiss Webster mice were injected subcutaneously with a conjugate vaccine adsorbed on aluminum hydroxide (Alhydrogel, Superfos, Denmark). Each mouse received 3 doses of 2 µg conjugated polysaccharide at days 0,28 and 42. The mice were bled at days 0, 28, 38 and 52.

### GYMP-specific antibodies by ELISA:

GYMP-specific IgG titers were estimated by ELISA using LMW GYMP (either OAc or dOA) linked to human serum albumin as the coating antigen.

The menY PS-specific IgG titers (geometric mean) elicited by the GYMP-TT conjugates after one and two injections are represented in Figure 1. They were measured by ELISA using dOA GYMP-HSA as a coating antigen. As can be seen in Figure 1, both types of GYMP conjugates (OA and dOA) generated similar levels of GYMP-specific IgG, suggesting that the OA group on the PS is not critical for immunogenicity. Figure 2 and Figure 3 show the ELISA binding of OA and dOA GYMP-TT antiserum (day 38) on OA and dOA GYMP-HSA coated plates respectively. On both antigen-coated plates the anti-dOA GYMP-TT antiserum react slightly better than the corresponding OA conjugate antiserum indicating that the OA on the PS is not necessary for immunogenicity.

### Serum bactericidal activity (SBA):

Serum bactericidal activity of sera was tested using the GYM strains described in Table 1, expressing polysaccharides with various levels of OA. Figure 4 shows SBA titers, measured with rabbit complement and OA menY strain 3790 of mouse antiserum induced with OA and dOA GYMP-TT conjugates. As shown in Figure 4, the dOA GYMP conjugates induced levels of SBA against menY strain 3790 (which expresses an OA capsular PS) similar to the corresponding OA conjugates, indicating that the OA group on the PS is not critical for immunogenicity and potency.

Furthermore the antibody induced with dOA GYMP conjugate is able to kill GYM strains which expresses capsular PS with various degrees of OA (Table 2).

**Table 2: Serum bactericidal activity (SBA) titers of dOA GYMP-TT conjugate antiserum (day 38) against menY strains**

| **Vaccine** | **GYM 3536^{b}** | **GYM1975^{b}** | **GYM 3790^{b}** |
|---|---|---|---|
| **dOA GYMP-TT^{a}** | 8,200 | 5,000 | 6,000 |
| **PBS** | <50 | <50 | <50 |

| | | | |
|---|---|---|---|
| ^{a} CD1 female mice received 2 doses of conjugate vaccine at days 0 and 28. Sera were obtained 10 days after the 2^{nd} injection at day 38. ^{b} GYM 3536 contains 52% OA on its PS, GYM 3790 has 90% and GYM 1975 has no OA. | | | |

### EXAMPLE 4: SPECIFICITY OF CONJUGATE VACCINE-INDUCED ANTIBODIES, ROLE OF THE OA GROUP ON THE POLYSACCHARIDE

### Specificity of binding of conjugate vaccine-induced antibodies:

Competitive inhibition of ELISA binding of vaccine-induced antibodies (the vaccine containing an OA GYMP conjugate or a dOA GYMP conjugate) to an OA GYMP HAS conjugate or to a dOA GYMP-HSA conjugate was carried out using high molecular weight (HMW) GYMP inhibitors containing sialic acids OA predominantly at C9 (GYM Slaterus) or at C7 (GYM S3790) or no OA at all (GYM S1975).

Figure 5 shows the specificity of dOA GYMP conjugate antiserum relative to dOA GYMP, OA GYMP predominantly at C7 and OA GYMP predominantly at C9. All three polysaccharide inhibitors are able to completely inhibit binding of these antiserum to OA GYMP HSA coated plates. OA GYMP (C9) is the least effective, indicating that the antibodies raised against the dOA GYMP conjugate do not really discriminate between OA and dOA polysaccharide.

Figure 6 shows the specificity of dOA GYMP conjugate antiserum relative to the same polysaccharide inhibitors described above but this time using binding to a dOA GYMP HSA coated plate. Here again, the polysaccharide inhibitors are able to completely inhibit binding, with the dOA polysaccharide being the best inhibitor.

Figure 7 shows the specificity of OA GYMP conjugate antiserum using an OA GYMP HSA coated plate. The three polysaccharide inhibitors are able to fully inhibit binding of antibodies to the coated plate, but in this case the OA GYMP at C9 seems to be the best inhibitor followed by dOA GYMP and then OA GYMP at C7, perhaps suggesting that the OA conjugate raised antibodies with epitope specificity increasing slightly towards a GYMP OA at C9. However, these antibodies recognize dOA epitopes in a very significant manner.

Figure 8 examines the specificity of anti-OA GYMP conjugate antiserum binding to a dOA GYMP HSA coated plate. The three Polysaccharide inhibitors are able to fully inhibit binding of this antiserum to the coated plate with dOA and OA GYMP at C7 are the best and equally potent inhibitors.

Collectively, these data indicate that in mice a dOA form of the GYMP conjugate will induce antibodies that will recognize equally well both OA and dOA GYM polysaccharides (Figure 5 and 6 respectively).

### Specificity of anti GYMP functional antibodies, role of the OA:

The specificity of functional antibodies induced by OA or dOA GYMP conjugates was examined by competitive inhibition of SBA (using rabbit complement) to an OA GYM strain (3790) with soluble GYMP inhibitors, having OA on their sialic acids predominantly at C7 or C9 or not at all.

Figure 9 shows the results of such inhibition using a mouse anti-dOA GYMP conjugate antiserum. The three polysaccharide inhibitors are able to completely inhibit the killing of strain 3790, with the dOA PS being roughly 5 times (at 50%) more effective than both OA polysaccharide inhibitors, which seem to inhibit in a similar fashion to each other. These data indicate that, on the surface of GYM live organisms expressing an OA polysaccharide, the antibodies against dOA GYMP recognize either a polysaccharide epitope containing dOA sialic acid (ca. 10 % on the polysaccharide) or that the antibodies raised are of higher affinity.

Figure 10 shows the results of a competitive inhibition of SBA using a mouse anti-OA GYMP conjugate antiserum. As for the dOA GYMP conjugate antiserum, dOA GYMP is the best polysaccharide at inhibiting the SBA to GYM 3790 organisms, followed by the other two OA GYMPS. These data strongly suggest that OA groups on the polysaccharide are not critical for the protective epitope to be recognized by functional antibodies.

### EXAMPLE 5: DETERMINATION OF THE SIZE OF THE GYMP EPITOPE

### Generation of oligosaccharide inhibitors of discrete length (1 to 9 repeats) from dOA GYMP:

The dOA GYMP was partially hydrolysed with 0.1N sodium acetate buffer pH 3 at 80 °C over a 2 hour time. The generated oligosaccharides of discrete length (1-9 repeating units) were then separated by size exclusion chromatography on a Superdex G-30 (Pharmacia) column. The elution of the oligosaccharides was monitored by UV detection at 214 nm. Fractions containing each individual oligosaccharide of discrete length were combined and freeze dried. The purity and size of the inhibitors thus obtained was performed by H-NMR spectroscopy at 600 MHz. Each GYM oligosaccharide inhibitor has a reducing beta sialic acid at its reducing end and α-D-glucose at the non-reducing other end.

### Competitive inhibition by ELISA using the oligosaccharide inhibitors:

Determination of the size of the GYMP epitope was achieved by competitive inhibition (ELISA) of binding between GYMP-specific rabbit antibody and GYMP-HSA coated plate with GYM oligosaccharides of increasing length.

Figure 11 represents an ELISA inhibition of a rabbit anti whole GYM antibody (DIFCO, Maryland) binding to a dOA GYMP-HSA coated plate, with dOA GYM oligosaccharides ranging from 1 to 10 repeating units. As can be seen on this figure, 1 and 2 repeating units are poor inhibitors of the binding while the 3 repeating units oligosaccharide (6 glycosyl residues) is able to completely inhibit the binding. These data indicates that the binding site of the antibody presumably makes contact with 5 sugar residues, since the 6^{th} residue, the reducing sialic acid, is in its beta conformation with the carboxylate group in an equatorial position. Increasing the size of the oligosaccharide (from 4 to 10 RUs) increases the inhibition on a mole per mole basis, suggesting a stabilization of the epitope conformation recognized by the antibody with increasing length.

Figure 12 represents inhibition of the same antibody binding on an OA GYMP-HSA coated plate with the same inhibitors. The pattern of inhibition is similar to the one observed in Figure 11, with increased binding beyond a 3 RUs oligosaccharide structure. These data have implications for the design of a GYMP conjugate, because they suggest that the minimum size of the GYMP hapten to be conjugated to carrier protein should be 10 repeating units.

### EXAMPLE 6 : POTENCY OF MENINGOCOCCAL Y CONJUGATE VACCINES IN COMBINATION

A combination of dOA GYMP-TT in combination with dOA GCMP-TT and dOA GWMP rPorB conjugate vaccines was tested for potency in Swiss Webster mice. The animals received subcutaneously 2 µg of each polysaccharide conjugated antigen either in combination or as stand-alone at days 0, and 28. They were bled at days 0, 28 and 38 and sera were analysed for SBA against GYM strain 3790 expressing OA GYMP.

Figure 13 shows the results of such an experiment. The SBA against GYM in the combination conjugate antiserum was not significantly different from that measured in the stand-alone GYMP conjugate antiserum, indicating that no immunological interference between the 3 conjugates was observed. Similarly no immunological interference was observed in the SBA arising from the GCMP and GWMP conjugate components whether in combination or stand-alone conjugates.

Further aspects of the invention are defined in the following numbered clauses.
1. An immunogenic conjugate comprising group Y meningococcal polysaccharide covalently coupled to polymeric carrier, including O-deacetylated O-acetyl-positive group Y meningococcal polysaccharide or a fragment thereof, wherein the degree of de-O-acetylation is greater than 80%, for use as a vaccine against N. meningitidis infection.
2. An immunogenic conjugate of clause 1, characterized in the degree of de- O-acetylation is 100%.
3. A polysaccharide according to clause 1 with a Molecular weight average of one selected from the group consisting of 10 kDa, 50kDa and 150 kDa.
4. A polysaccharide according to clause 1 or clause 2 that has been fragmented and wherein the size of the fragment contains between 5 repeating units (ca 2.5 kDa) and 200 repeating-units (ca 100 kDa).
5. A polysaccharide according to clause 1 or clause 2 that has been fragmented and wherein the size of the fragment contains between 20 repeating units (ca 10 kDa) and 40 repeating units (ca 20 kDa).
6. A conjugate product comprising a de-O-acetylated meningococcal Y polysaccharide conjugated to a carrier protein.
7. A conjugate product according to clause 4, wherein the carrier protein is a bacterial toxin or toxoid.
8. A conjugate product according to clause 5, wherein the bacteria toxin or toxoid is selected from the group consisting of diphtheria, tetanus, pseudomonas, staphylococcus, streptococcus, pertussis and *Escherichia coli* toxin or toxoid.
9. A conjugate product according to clause 6, wherein the bacterial toxin or toxoid is tetanus toxin or toxoid.
10. A conjugate product, wherein the modified meningococcal Y polysaccharide is as defined in clause 2.
11. A vaccine comprising a conjugate product as defined in clause 4.
12. A vaccine according to clause 9, wherein the bacterial toxin or toxoid is selected from the group consisting of diphtheria, tetanus, pseudomonas, staphylococcus, streptococcus, meningococcal porin B, pertussis and Escherichia coli toxin or toxoid.
13. A vaccine according to clause 10, wherein the bacterial toxin or toxoid is tetanus toxin or toxoid.
14. A vaccine according to clause 1, which comprises an adjuvant.
15. A vaccine according to clause 12, wherein the adjuvant is aluminum hydroxide.
16. A vaccine according to clause 9, which is adapted for administration by injection.
17. A vaccine according to clause 9, wherein the conjugated material comprises a polysaccharide as defined in clause 2.
18. The use of a modified polysaccharide as defined in clause 1 in the manufacture of a vaccine for use in meningitides against Group Y Neisseria meningitides.
19. The use of a conjugated material as defined in clause 4 in the manufacture of a vaccine for use in meningitides against Group Y Neisseria meningitides.
20. A process for the manufacture of a vaccine for use in immunisation against Group Y Neisseria meningitides, which process comprises providing a modified polysaccharide as defined in clause 1 and optionally mixing it with one or more of a pharmaceutically acceptable carrier medium, diluent or adjuvant.
21. A process for the manufacture of a vaccine for use in immunisation against Group Y Neisseria meningitides, which process comprises providing a conjugated material as defined in clause 4 and optionally mixing it with one or more of a pharmaceutically acceptable carrier medium, diluent or adjuvant.
22. The use of a vaccine as defined in clause 9 for meningitides against Group Y Neisseria meningitides.
23. A process for vaccinating a warm-blooded animal against Group Y Neisseria meningitides, which process comprises administering a vaccine as defined in clause 9 to the animal.
24. A process for the preparation of a modified meningococcal Y polysaccharide, which process comprises subjecting a meningococcal Y polysaccharide to base hydrolysis such that the meningococcal Y polysaccharide is at least in part de-O-acetylated.
25. A process for the preparation of a modified meningococcal Y polysaccharide, which process comprises subjecting a meningococcal Y polysaccharide to acid hydrolysis such that the meningococcal Y polysaccharide is fragmented.
26. A process for the preparation of a modified meningococcal Y polysaccharide fragment having a molecular weight of from 10 to 20 kDa, which process comprises: (a) providing an at least partially purified meningococcal Y polysaccharide; (b) base hydrolysis of the polysaccharide; (c) acid hydrolysis of the product of step (a); and optionally (d) re-N-acetylating of the product of step (b).
27. A process for producing a conjugated product as defined in clause 4, which process comprises contacting a modified meningococcal Y polysaccharide with a carrier protein, optionally in the presence of a coupling agent.
28. A combination meningococcal conjugate vaccine including de-OAc forms of group Y, group C and group W135 meningococcal polysaccharides for prevention of meningococcal Y, C and W135 disease.

## Claims

1. An immunogenic conjugate comprising a carrier protein and a group Y meningococcal polysaccharide fragment obtained from a native O-acetyl-positive group Y meningococcal polysaccharide,
i. wherein the polysaccharide fragment has been de-O-acetylated by base hydrolysis and is completely N-acetylated;
ii. wherein the carrier protein is covalently coupled to the polysaccharide fragment at a sialic acid exocyclic side chain of the polysaccharide fragment; and
iii. wherein the immunogenic conjugate is suitable for use as a vaccine against Group Y *N. meningitidis* infection.

2. The immunogenic conjugate as defined in claim 1, wherein said polysaccharide fragment has a molecular weight average selected from the group consisting of 10 kDa, 50 kDa, and 150 kDa.

3. The immunogenic conjugate as defined in claim 1, wherein said polysaccharide fragment has a molecular weight of less than 100 kDa, or a molecular weight from about 2.5 kDa to about 100 kDa, from 5 kDa to 50 kDa, or from about 10 kDa to about 20 kDa.

4. The conjugate as defined in any one of the preceding claims, wherein the carrier protein is a bacterial toxin or a bacterial toxoid.

5. The conjugate as defined in claim 4, wherein the bacterial toxin or the bacterial toxoid is selected from the group consisting of a diphtheria toxin, a diphtheria toxoid, a tetanus toxin, a tetanus toxoid, a pseudomonas toxin, a pseudomonas toxoid, a staphylococcus toxin, a staphylococcus toxoid, a streptococcus toxin, a streptococcus toxoid, a meningococcal porin B, a pertussis toxin, a pertussis toxoid, an *Escherichia coli toxin,* and an *Escherichia coli* toxoid.

6. A vaccine comprising the immunogenic conjugate as defined in any one of the preceding claims and a pharmaceutically acceptable carrier medium, excipient, or diluent.

7. The vaccine as defined in claim 6, wherein the carrier protein is a bacterial toxin or a bacterial toxoid selected from the group consisting of a diphtheria toxin, a diphtheria toxoid, a tetanus toxin, a tetanus toxoid, a pseudomonas toxin, a pseudomonas toxoid, a staphylococcus toxin, a staphylococcus toxoid, a streptococcus toxin, a streptococcus toxoid, a meningococcal porin B, a pertussis toxin, a pertussis toxoid, an *Escherichia coli* toxin, and an *Escherichia coli* toxoid.

8. The vaccine as defined in claim 6 or 7, further comprising an adjuvant, preferably wherein the adjuvant is aluminum hydroxide.

9. A vaccine as defined in any of claims 6-8, which is adapted for administration by injection.

10. A vaccine as defined in any of claims 6-9 for use in vaccinating a warm-blooded animal against Group Y *Neisseria meningitidis.*

11. The use of the immunogenic conjugate as defined in any one of claims 1-5 in the manufacture of a vaccine for use against Group Y *Neisseria meningitidis.*

12. A process for the manufacture of a vaccine for use in immunisation against Group Y *Neisseria meningitidis,* which process comprises providing a conjugate as defined in any of claims 1-5 and optionally mixing it with one or more of a pharmaceutically acceptable carrier medium, excipient, diluent, or adjuvant.

13. A process for the preparation of a modified meningococcal Y polysaccharide fragment obtained from a native O-acetyl-positive group Y meningococcal polysaccharide,
i. wherein the modified polysaccharide fragment has been de-O-acetylated by base hydrolysis and is completely N- acetylated, which process comprises:
(b) providing an at least partially purified native O-acetyl positive meningococcal Y polysaccharide;
(c) conducting base hydrolysis of the polysaccharide such that the polysaccharide is at least partially de-O-acetylated;
(d) conducting acid hydrolysis of the product of step (b) such that the de-O-acetylated polysaccharide is fragmented; and
(e) completely re-N-acetylating the product of step (c).

14. A process for producing the immunogenic conjugate as defined in any of claims 1-5, which process comprises contacting the modified meningococcal Y polysaccharide fragment prepared according to the process of claim 13 with a carrier protein, optionally in the presence of a coupling agent to couple said modified meningococcal Y polysaccharide fragment to said carrier protein to form said conjugate.

15. A combination meningococcal conjugate vaccine comprising de-O-acetylated forms of group Y, group C, and group W135 meningococcal polysaccharides for prevention of disease caused by meningococcal Y, C, and W135, wherein the de-O-acetylated group Y form is comprised within a conjugate as defined in any of claims 1-5.
